# EUROPEAN PATENT APPLICATION

(11) **EP 1 493 824 A1**
(43) Date of publication of application: **05.01.2005**
(21) Application number: 03291624.9
(22) Date of filing: 02.07.2003
(51) Int. Cl.: C12Q 1/68

(54) **Method for detection of mutations in DNA**

(71) Applicant: Consortium National de Recherche en Genomique (CNRG), 91000 Evry (FR)
(72) Inventor: Gutivo, Glynne, 75014 Paris (FR); Mauger, Florence, 91000 Evry (FR)
(74) Representative: Warcoin, Jacques

(57) **Abstract**

A method for the detection in a given DNA sequence of DNA mutations, single nucleotide polymorphisms, and insertions and deletions, said method comprising the step of a) producing replicate(s) with an engineered polymerase of said given DNA sequence with part or all of at least one of the four natural DNA bases exchanged against a not natural base; b) using said not natural base to cleave the replicate(s) obtained in step a) and to produce a DNA product presenting sequence-specific fragments; c) analyzing said sequence-specific fragments obtained in step b) by mass spectrometry to get sequence-specific fragment patterns; and d) using the sequence-specific fragment patterns obtained in step c) to identify sequence changes relative to a reference to said given DNA sequence. A kit for the detection of DNA mutations, single nucleotide polymorphisms, and insertions and deletions.

## Description

The present invention relates to a method for the detection in a given DNA sequence of DNA mutations, single nucleotide polymorphisms (SNP), and insertions and deletions (indels), and a kit for said detection.

The most important of the genome projects, the complete sequence of the human genome, is now finished. This project reveals the complete sequence of the 3 billion bases and the relative positions of all estimated 100.000 genes in this genome. Having this sequence opens unlimited possibilities for the elucidation of gene function and interaction of different genes. In recent years a systematic effort (SNP consortium) has been underway to identify SNPs throughout the human genome and so far several million of these differences between different human beings have been identified. However, it is assumed that a significant number of SNPs both frequent and rare have been missed.

For cost reasons it is inconceivable to continue the detection of SNPs with the currently employed strategies, for example standard DNA sequencing. Also projects where mutations are randomly introduced into organisms require efficient methods for identifying the nature and position of mutations.

Some methods to genotype previously identified mutations, SNPs, or indels has been proposed. Among such methods, efficient method such as matrix-assisted laser desorption/ionization time-of-flight mass spectrometry (MALDI) has revolutionized the mass spectrometric analysis of biomolecules (Karas M. & Hillenkamp F. *Anal. Chem*. **60**, 2299-2301 (1988)). The field of DNA analysis by mass spectrometry was recently extensively reviewed by Tost and Gut (Mass Spectrometry Reviews, **21**, 388-418 (2002)) and Sauer and Gut (Journal of Chromatography B, **782**, 73-87, (2002)). MALDI has been applied to the analysis of DNA in variations that range from the analysis of PCR products to approaches using allele specific termination to single nucleotide primer extension reactions and sequencing (Liu Y.-H. *et al*., *Rapid Commun*. *Mass Spectrom.* **9**, 735-743 (1995); Chang L.-Y. *et al*. *Rapid Commun. Mass Spectrom.* **9**, 772-774 (1995); Little D.P. *et al*. *J. Mol*. *Med*. **75**, 745-750 (1997); Haff L. & Smirnov I.P., *Genome Res.* **7**, 378-388 (1997), Fei Z.*et al*., *Nucleic Acids Res.* **26**, 2827-2828 (1998); Ross *P.et al*., *Nature Biotech.* **16**, 1347-1351 (1998);Ross P.L. *et al*., *P., Anal. Chem.* **69**, 4197-4202 (1997); Griffin. T.J.*et al*., L.M. *Nature Biotech.* **15**, 1368-1372 (1997); Köster H. *et al*., US Patent 6,043,031). Spin column purification and/or magnetic bead technology, reversed-phase purification, or ion-exchange resins are frequently applied prior to mass spectrometric analysis.

Most methods for DNA analysis with mass spectrometric detection have been applied to the analysis of known mutations or SNPs (Tost and Gut, Mass Spectrometry Reviews, **21**, 388-418 (2002)). Analysis of known mutations, SNPs and indels with the cleavage of the primer is taught by Montforte *et al*. (US 5,830,655 and US 6,566,055). The DNA positions that are tested therein are not immediately related with the cleavage position and cleavage functionalities are chemically synthesized into the primers.

Photocleavable DNA bases that are lodged in primer sequences are described in EP 0855403 and in DE 10108453.

Furthermore, only a few publications deal with the detection of so far unknown mutations or SNPs (Hahner *et al*,. Nucleic Acids Research, **25**, 1957-1964 (1997), Rodi *et al*., BioTechniques, **32**, S62-S69 (2002), Krebs *et al*., Nucleic Acids Research, **31**, e37 (2003)). In these publications a target sequence is amplified by PCR. One of the primers contains a transcription initiation site. In a second reaction the PCR product is transcribed into RNA. The RNA is cleaved with base-specific RNAses and the products analyzed by mass spectrometry. These methods require one DNA polymerase and one RNA polymerase reaction and an RNAse degradation. Due to the cost of all of these enzymes these procedures are very expensive.

US 6,468,748 has also proposed creating fingerprints of PCR products by sequence specific restriction, digestion by restriction endonucleases, and recording these by mass spectrometry.

Further methods has been proposed which describe chemical cleavage using chemistries similar to the ones used in chemical mismatch cleavage and Maxam-Gilbert sequencing. In US 5,217,863, chemical mismatch cleavage allows to detect unknown mutations with detection by gel migration of fluorescently or radioactively labeled DNA fragments. However, combining chemical mismatch cleavage with mass spectrometric detection has so far not been shown and is problematic due to the chemical attack on positions neighboring the mismatch and terminal nucleotides.

Pirrung and Zhao in US 6,294,659 have described a photocleavable nucleotide triphosphate that can be included sequence specifically into a primer extension reaction or PCR.

The Inventors thus assigned themselves the task of providing a new method for the detection of previously unknown mutations, known mutations, unknown and known SNPs, and unknown and known indels that is cost efficient, fast, accurate, highly reliable, easily automated, and so lends itself to high-throughput.

This objective is obtained by the following described method of detection.

The present invention relates to a method for the detection in a given DNA sequence of DNA mutations, SNPs, and indels comprising the steps of:
a) producing replicate(s) with an engineered polymerase of said given DNA sequence with part or all of at least one of the four natural DNA bases exchanged against a not natural base;
b) using said not natural base to cleave the replicate(s) obtained in step a) and to produce a DNA product presenting sequence-specific fragments;
c) analyzing said sequence-specific fragments obtained in step b) by mass spectrometry to get sequence-specific fragment patterns; and
d) using the sequence-specific fragment patterns obtained in step c) to identify sequence changes relative to a reference to said given DNA sequence.

Within the meaning of the present invention, the word "mutation" is to be taken as meaning previously unknown mutation or known mutation.

Nature has specifically evolved DNA polymerases to process DNA building blocks (dATP, dGTP, dCTP, and TTP) during replication. DNA polymerases are used for example in PCR. On the other hand reverse transcriptases have evolved to generate RNA on a polymerase reaction. The DNA and RNA replication machineries are very different and specifically geared to not use each others substrates. Thus NTPs are not suitable substrates for a DNA polymerase.

Within the meaning of the present invention, "engineered polymerase" is to be taken as meaning that substantial modifications of DNA polymerase have been made to make them process NTPs. Such engineered polymerases are made by targeted mutations or random mutations followed by selection.

Within the meaning of the present invention, "not natural base" is to be taken as meaning that the base used is usually not used and cannot be processed by an enzyme such as Taq-polymerase. Such a base can be another known base, for example NTP in place of dNTP, or chemically modified base. The man skilled in the art can imagine a modified base which must suit the engineering polymerase.

Within the meaning of the present invention, "part or all" is to be taken as meaning that, according to the aim searched, all of the one of the four natural bases are exchanged, or only part of those are exchanged during the replicate process.

The method of the present invention is a facile and dramatically more economic method for detecting mutation, SNP, and indel by integrating a cleavable base, for example an RNA base that results in backbone cleavage when the so replicated sample is treated with alkali. Fragment analysis can very effectively be carried out by mass spectrometry.

One more advantage of the method of the present invention is that an entire genome of one individual could be re-sequenced on one mass spectrometer in a matter of weeks.

The streamlined method of the present invention makes use of the potential of mass spectrometers to distinguish large numbers of products simultaneously in one spectrum and is able to record a single spectrum in a few seconds.

The method of the present invention consists of recording a fragment signature of a given stretch of DNA sequence by mass spectrometry and comparing it with a reference. Differences between two signatures are indicative of mutations, SNPs, and insertion/deletions. Due to the mass profile, the position and nature of the mutations can unambiguously be assigned. No additional sequence analysis is required. DNA is prepared from a template DNA molecule by a DNA replication method, such as PCR, during which chemically or enzymatically inducible nucleotides are incorporated. Sequence specific fragmentation of the target DNA is achieved by chemical or enzymatic cleavage. Triggering these nucleotides to cleave the DNA strand specifically results in the required fragments for mass spectrometric analysis. Fragment analysis provides a sequence specific fingerprint, which allows the identification of differences between a reference DNA sequence and an unknown sample.

According to one advantageous form of embodiment of the invention, the not natural base in step a) is selected from the group consisting of an RNA base (ATP, GTP, CTP, or UTP), a phosphorothioate base, a phosphoroselenoate base, a photochemically cleavage inducible base.

According to another advantageous form of embodiment of the invention, in the replicate obtained in step a), more than 50% of one of the four natural DNA bases is exchanged against a not natural base, more particularly more than 70%.

It is to be understood that the man skilled in the art can modifiy as desired the number of the base to be exchanged, and as well the proportion in the replicate obtained in step a).

According to another advantageous form of embodiment of the invention, in the replicate obtained in step a), 100% of one of the four natural DNA bases is exchanged against a not natural base.

According to another advantageous form of embodiment of the invention, the RNA base is cleaved in step b) by treatment with alkali and incubation at elevated temperature.

According to another advantageous form of embodiment of the invention, the UTP base is cleaved in step b) by uracil-deglycosylate followed by treatment with alkali, and optionally incubation at elevated temperature.

According to another advantageous form of embodiment of the invention, the phosphorothioate or phosphoroselenoate base is cleaved in step b) by condensation of a compound of the nature OH-(CH2)n-I , where n=2-5, and incubation at elevated temperature.

According to another advantageous form of embodiment of the invention, a photochemically cleavage inducible base is cleaved in step b) by exposure to light.

According to another advantageous form of embodiment of the invention, the step a) of producing replicate(s) is carried out with a procedure selected from the group consisting of the polymerase chain reaction (PCR) and the linear DNA copying procedure, more particularly a rolling circle replication.

According to another advantageous form of embodiment of the invention, the method comprises further a step a') between step a) and step b), wherein in step a') the sequence-specific fragments are purified, for example on reversed-phase material or with ion exchange resins.

According to another advantageous form of embodiment of the invention, the method comprises further a step b') between step b) and step c), wherein in step b') the sequence-specific fragments are purified, for example on reversed-phase material or with ion exchange resins.

In still another form of embodiment of the invention, the mass spectrometer used for step c) is a MALDI or an ESI mass spectrometer.

In the method according to the invention, a stretch of genomic DNA sequence is replicated by a procedure such as PCR. However, in contrast to regular PCR or other DNA replication systems, one or more of the four nucleotide substrates (dATP, dGTP, dCTP, or TTP) are substituted at least partially with modified nucleotide equivalent. Modified nucleotides are for example ribonucleotides (ATP, GTP, CTP, or UTP), phosphorothioates (α-S-dATP, α-S-dGTP, α-S-dCTP, or α-S-TTP), base modified nucleotides or similar. As it was previously said, the man skilled in the art can imagine new modified bases or nucleotides.

It is key to this invention that the modified nucleotides are readily and sequence-specifically incorporated by the DNA polymerase with similar kinetics as the regular substrates.

Further it is desirable that the DNA polymerase has some 3'-5' proof-reading ability to assure that the sequence is replicated perfectly. Also, exonuclease resistant bases can be included in the primer to prevent their degradation. These can be phosphorothioate bridges.

The PCR products containing the modified nucleotides are thereafter subjected to a treatment that results in base- and thus sequence-specific cleavage of the backbone at the positions of the modified nucleotides. The direct incorporation of modified substrates into the PCR is technically demanding and can be achieved by the application of engineered DNA polymerases.

As PCR is an exponential process, in which a new molecule is generated using a product from a previous round as template, it can be difficult to copy a molecule that already contains modification into another modification containing molecule. To alleviate this problem it is possible to run the PCR with regular substrates to generate a sufficient population of template. Residual nucleotides can then be destroyed enzymatically, for example with shrimp alkaline phosphatase. In a second round the set of nucleotides containing the modified nucleotide are added with the accordingly engineered DNA polymerase. By varying the amount of primers in the two reactions enrichment of one of the two DNA strands, preferably the strand that is to be analysed by mass spectrometry, can be achieved. Analysing the cleavage of only one strand reduces the complexity of mass spectra.

The cleavage chemistries are for example alkali hydrolysis for RNA containing PCR products; cleavage at positions of phosphorothioates by addition of compounds of the nature (OH-(CH2)n-I, where n = 2-5) followed by heat treatment; photochemical cleavage using a photocleavable base; cleavage by enzymatic removal of UTP by uracil-deglycosylase to create an abasic site followed by alkali treatment.

The preferred substrate to carry out alkali treatment is NH₄OH (ammoniumhydroxide). This compound is well tolerated by the ensuing mass spectrometric analysis and reduces problems of signal suppression and alkali adducts.

The step a') or b') of a purification could be carried out on reversed-phase material such as ZipTips (Millipore), or with ion exchange resins to remove salts that would reduce the resolution in the mass spectrometric analysis. Optional purification can take place either after the enzymatic steps or after the fragmentation step.

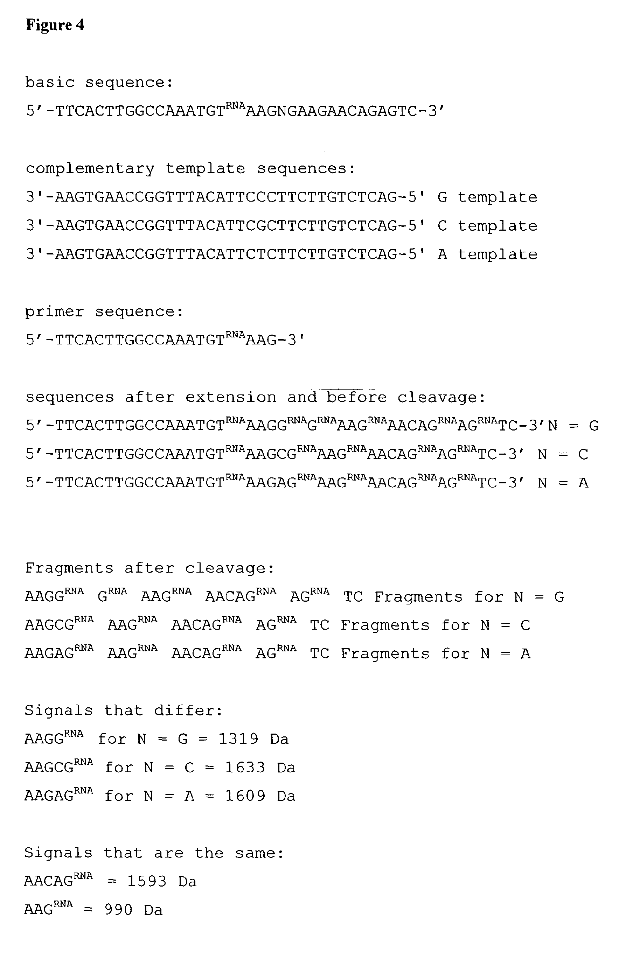

Mass spectrometry analysis is preferably done by MALDI. However, electrospray ionisation mass spectrometry would also be suitable. The mass signatures are used to reassemble the target sequence. Deviations from the baseline target sequence can be identified by different manifestations as outlined in Figure 4), a) a base change involving the base that is exchanged in the polymerase reaction results in the appearance or disappearance of a peak; b) a change in between two cleavage positions results in a shift of that peak due to the base substitution.

In the method according to the invention, RNA containing PCR products are preferably produced directly, subjected to alkali cleavage and analysed in the mass spectrometer. Further two or more independent reactions are carried out, in which always another base is replaced. Thus the patterns of different substitutions can be combined for the verification of the tested sequence in relation to a reference sequence. Deviations are interpreted according to the demonstration in Figure 4.

Another object of the present invention is a kit for the detection in a given DNA sequence of DNA mutations, single nucleotide polymorphisms, and insertions and deletions for implementing a method according to claim 1 comprising:
- An engineered DNA polymerase,
- A set of non-natural bases and dNTPs,
- A buffer.

Apart from the above arrangements, the invention also includes other arrangements which will emerge from the following description, which refers to examples of embodiments of the method of the present invention, as well as to the annexed drawings, in which:

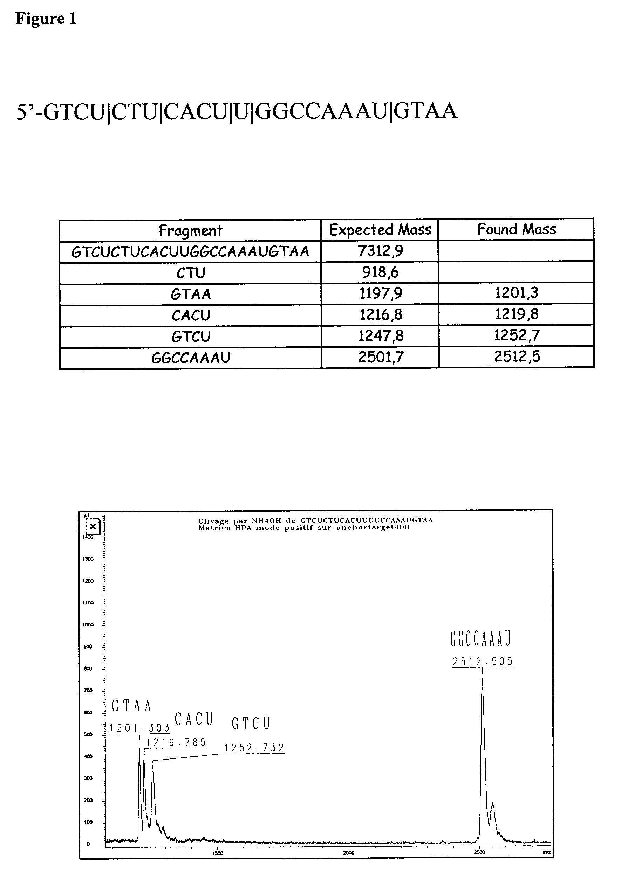

- Figure 1 illustrates a synthetic sequence of 24 bases with 5 uracil bases which is subjected to treatment with NH₄OH. All potential cleavage products larger than 3 bases are found and can unambiguously be assigned to fragments. The cleavage products is analysed by MALDI.

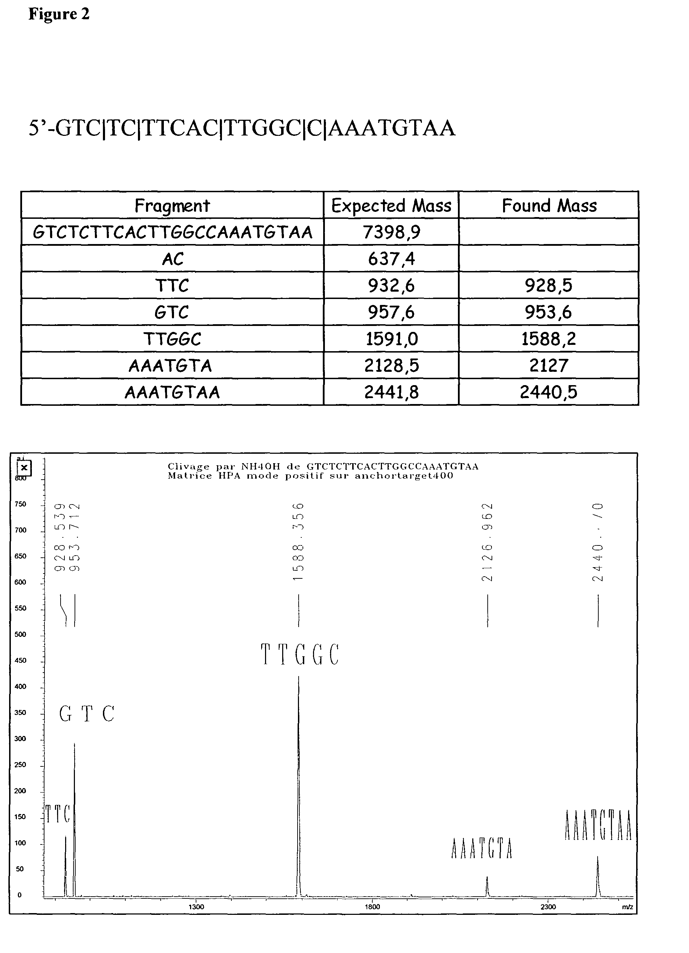

- Figure 2 illustrates a synthetic sequence of 24 bases with 5 cytosine (RNA) bases which is subjected to treatment with NH₄OH. All potential cleavage products larger than 2 bases are found and can unambiguously be assigned to fragments. The peak at 2127 Da corresponds to a spurious fragment (shortened) present before cleavage that does not correspond to a real cleavage product.

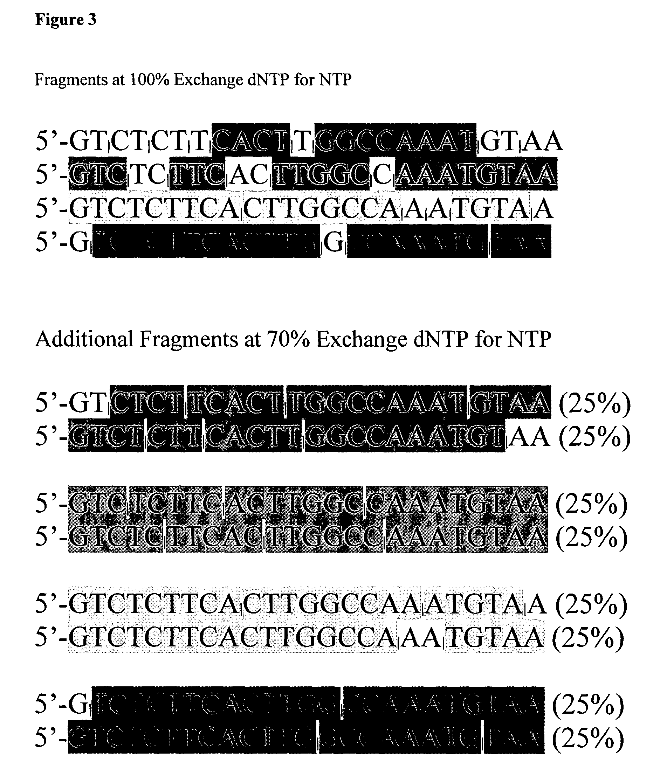

- Figure 3 illustrates theoretical overlap of fragment patterns of a single sequence. Always one of the four bases is exchanged by a RNA base and site specifically cleaved. This demonstrates that at 100% exchange of two bases (recording two lanes) sufficient sequence coverage is achieved for the identification of a change in the sequence.
- Figure 4 illustrates enzymatic extension of a primer on synthetic templates with different sequences. The primer contains a RNA base on the forth base from the 3'-end. Thus a major part of the primer is cleaved off during the NH₄OH treatment. In this example a immediate termination at the first base is observed (peaks 1528 Da in the C track and 1552 Da in the A track). These peaks are identifiable by the lack of the 3'-terminal phosphate group. This is due to the DNA polymerase falling off. However, once the polymerase has started moving this problem decreases, as can be seen from the fragment AACAG at 1593 Da and the fragment AGG at 990 Da, where corresponding products lacking the phosphate groups are not found. These initial termination fragments are consistently found in all traces. Note: The primer contributes a non RNA G. Consequently cleavage does not take place at this base.
   It should be clearly understood, however, that these examples are given solely by way of illustration of the object of the invention, of which they in no way constitute a limitation.

### Example 1 : general implementation

A template DNA, two primers (containing a single phosphorotioate bridge near the 3'-end), dATP, dGTP, dCTP, UTP, buffer, magnesium, and an engineered DNA polymerase are used to amplify a 50-1000 base stretch of DNA with 30 cycles of a temperature profile of 55°C for 15 seconds, 72°C for 30 seconds, 95°C for 15 seconds. The PCR product is treated with 25 M NH₄OH (total pH = 11) for 1 hour at 55°C. Thereafter the solution is desalted either on ZipTip reversed-phase columns (Millipore) or by the addition of ionexchange resin and transferred onto the MALDI target. The target can be pre-coated with matrix, alternatively the matrix solution is mixed with the samples and transferred to the MALDI target surface to dry. The MALDI target is introduced into a MALDI mass spectrometer and analysed. The mass spectra show complex peak patterns that correspond to fragments of the whole PCR product (see Figure 1, 2 and 4).

### Example 2: Resequencing of a candidate fragment with one amplification step PCR:

We used forward primer (3.75 pmol), 3.75 pmol reverse primer,1 µl 10x PCR buffer (UP2, pH 8.8), 1 µl MgCl₂ (20 mM), 0.125 µl each dCTP, dATP, dGTP and UTP (2 mM each), 0.25 µl engineered DNA polymerase (5U/ul) fill to 10 µl with water.

Cycling is carried out as follows:
1.95°C 2 min,
2. 95°C 20 sec,
3. 68°C 30 sec,
4. 72°C 30 sec,
steps 2-4 are repeated 35 times.

Optionally the PCR product can be purified at this stage. Possible methods are ethanol precipitation, reversed-phase purification or similar.

### Cleavage:

25 M NH₄OH is added to the PCR product and incubated for 1 hour at 55°C. Alkali cleavage of RNA bases happens on the 3' side of the phosphate group. Due to this it is easy to tell the difference between a premature termination of the DNA polymerase extension reaction and the cleavage product (as the cleavage product is heavier by the phosphate group (see Figure 4).

### MALDI analysis:

0.5 µl of saturated 3-HPA matrix is mixed with 0.5 µl of the sample and deposited on the MALDI target surface. Analysis is carried out either in positive or negative ion mode on a MALDI mass spectrometer.

It is not necessary that the PCR is carried out with a 100% replacement of one modified nucleotide triphosphate.
However more than 70% should be replaced, so that the probability for each N_{modified} - N_{non-modified} fragment is more than 50%. Fragments with N_{modified} - N_{modified} - N_{modified} can be used in addition for the sequence assignment and products are in the higher mass range. Further sequencially T, C, G, and A can replaced individually in this procedure. Each replacements results in entirely different fingerprints (Figure 3). The joined information of two different base exchanges is usually sufficient for the entire sequence interpretation of double stranded DNA. Thus two reactions are sufficient for double stranded re-sequencing. This corresponds to what is required for fluorescent dye terminator sequencing.

### Example 3: Resequencing of a candidate fragment with a pre-amplification step

### PCR:

We use forward primer (0.5 pmol), 3.75 pmol reverse primer,1 µl 10x PCR buffer (UP2, pH 8.8), 1 µl MgCl₂ (20 mM), 0.125 µl each dCTP, dATP, dGTP and dTTP (2 mM each), 0.25 µl Taq-polymerase (5U/ul) fill to 10 µl with water.
Cycling is carried out as follows:
1. 95°C 2 min,
2. 95°C 20 sec,
3. 68°C 30 sec,
4. 72°C 30 sec,
steps 2-4 are repeated 25 times.

### Cleavage :

Shrimp alkaline phosphatase digest is carried out with 0.5 µl SAP (1U/ul) which was added and the reaction is incubated at 37°C for 37 min, then the SAP is denatured at 90°C for 10 min.

### Copy:

We use forward primer (20 pmol), 0.125 ul each dCTP, dATP, dGTP and UTP (0.02 mM each), 0.25 µl engineered DNA polymerase (5U/ul) fill to 12 µl with water.

Cycling is carried out as follows:
1. 95°C 2 min,
2. 95°C 20 sec,
3. 68°C 30 sec,
4. 72°C 30 sec,
steps 2-4 are repeated 35 times.

### Cleavage:

0.5 M NH₄OH is added to the PCR product and incubated for 1 hour at 55°C.

### MALDI analysis:

0.5 µl of saturated 3-HPA matrix is mixed with 0.5 µl of the sample and deposited on the MALDI target surface. Anchor targets with 400 µm anchors are preferably used. Analysis is carried out either in positive or negative ion mode on a MALDI mass spectrometer.

### Example 4: Whole genome resequencing strategy

Pure genomic DNA from one individual is digested with a frequent cutter restriction enzyme (e.g. EcoRI). Alternatively, whole genomic DNA can be broken into unspecific small clonable fragments with the help of ultrasound. The fragments are cloned into a vector and cells are transfected with the vector. Cells surviving the procedure are plated, grown and colonies picked (this procedure is done in analogy to the procedure used in Celniker et al., Genome Biology, 3, 1-14 (2002). Two primers specific for the cloning vector are used to PCR amplify the cloned insert (according to Example 1 or 2). Individual results are used to bioinformatically assign sequence differences between the human reference sequence and the analysed individual.

Examples of cleavage profiles are shown in Figures 1, 2, and 4.

## Claims

1. Method for the detection in a given DNA sequence of DNA mutations, single nucleotide polymorphisms, and insertions and deletions comprising the steps of:
a) producing replicate(s) with an engineered polymerase of said given DNA sequence with part or all of at least one of the four natural DNA bases exchanged against a not natural base;
b) using said not natural base to cleave the replicate(s) obtained in step a) and to produce a DNA product presenting sequence-specific fragments;
c) analyzing said sequence-specific fragments obtained in step b) by mass spectrometry to get sequence-specific fragment patterns; and
d) using the sequence-specific fragment patterns obtained in step c) to identify sequence changes relative to a reference to said given DNA sequence.

2. Method according to claim 1 wherein the not natural base in step a) is selected from the group consisting of an RNA base (ATP, GTP, CTP, or UTP), a phosphorothioate base, a phosphoroselenoate base, a photochemically cleavage inducible base.

3. Method according to claim 1 and 2 wherein in the replicate more than 50% of one of the four natural DNA bases is exchanged against a not natural base, more particularly more than 70%.

4. Method according to claim 3 wherein in the replicate 100% of one of the four natural DNA bases is exchanged against a not natural base.

5. Method according to claim 2 wherein the RNA base is cleaved in step b) by treatment with alkali and incubation at elevated temperature.

6. Method according to claim 2 wherein the UTP base is cleaved in step b) by uracil-deglycosylate followed by treatment with alkali.

7. Method according to claim 2 in which the phosphorothioate or phosphoroselenoate base is cleaved in step b) by condensation of a compound of the nature OH-(CH2)n-I , where n=2-5, and incubation at elevated temperature.

8. Method according to claim 2 in which a photochemically cleavage inducible base is cleaved in step b) by exposure to light.

9. Method according to claim 1 wherein the step a) of producing replicate(s) is carried out with a procedure selected from the group consisting of the polymerase chain reaction (PCR) and the linear DNA copying procedure.

10. Method according to claim 9 wherein the linear copying procedure is a rolling circle replication.

11. Method according to claim 1 comprising further a step a') between step a) and step b), wherein in step a') the sequence-specific fragments are purified, for example on reversed-phase material or with ion exchange resins.

12. Method according to claim 1 comprising further a step b') between step b) and step c), wherein in step b') the sequence-specific fragments are purified, for example on reversed-phase material or with ion exchange resins.

13. Method according to claim 1 wherein the mass spectrometer used for step c) is a MALDI or an ESI mass spectrometer.

14. Kit for the detection in a given DNA sequence of DNA mutations, single nucleotide polymorphisms, and insertions and deletions for implementing a method according to claim 1 comprising:
• An engineered DNA polymerase,
• A set of non-natural bases and dNTPs,
• A buffer.
